# FASCICULE DE BREVET EUROPEEN

(11) **EP 2 950 876 B1**
(45) Date de publication et mention de la délivrance du brevet: **15.04.2020**
(21) Numéro de dépôt: 14704617.1
(22) Date de dépôt: 28.01.2014
(51) Int. Cl.: A61M 39/10, A61M 39/26

(54) **CONNECTEUR À USAGE MÉDICAL PERFECTIONNÉ**
VERBESSERTER MEDIZINISCHER VERBINDER
IMPROVED MEDICAL CONNECTOR

(30) Priorité: 31.01.2013 FR 1350836
(43) Date de publication de la demande: 09.12.2015
(73) Titulaire: CAIR L.G.L., 69380 Lissieu (FR)
(72) Inventeur: LOPEZ, Georges Antoine, 69130 Ecully (FR); DELORME, Patrick, 69630 Chaponost (FR)
(74) Mandataire: Cabinet Laurent & Charras
(86) Numéro de dépôt international: PCT/FR2014/050153
(87) Numéro de publication internationale: WO 2014/118462

(56) Documents cités:
- EP-A1- 0 798 013
- WO-A1-2006/013433
- WO-A2-2008/052140
- FR-A1- 2 894 150

## Description

La présente invention se rapporte au secteur technique des connecteurs à usage médical, et concerne plus particulièrement un connecteur à usage médical du type comprenant une chambre solidaire d'un raccord muni d'une aiguille pour la distribution d'un fluide, l'aiguille étant gainée dans un joint élastique.

### ART ANTERIEUR

On connait du document EP 1 890 760 un connecteur à usage médical présentant une première extrémité dite « extrémité amont », destinée à être connectée à un cathéter relié à un patient; et une seconde extrémité, désignée « extrémité avale », destinée à coopérer avec un dispositif de prélèvement ou d'injection de liquide par le biais d'un connecteur du type luer mâle. Dans la suite, on se réfèrera aux expressions « extrémité amont » et « extrémité avale », quel que soit le sens de circulation du liquide.

En pratique, ce connecteur présente une chambre solidaire à sa base d'un raccord constituant l'extrémité amont du connecteur proprement dit. L'extrémité libre de la chambre, à l'opposé de la base, est destinée à recevoir, par friction, l'embout d'un connecteur du type luer mâle. Le passage du liquide entre le cathéter connecté à l'extrémité amont du connecteur et l'extrémité du luer mâle est assuré par le biais d'une aiguille solidaire du corps du raccord. L'aiguille s'étend dans la chambre et débouche dans l'extrémité terminale de ladite chambre.

L'aiguille est gainée et maintenue dans l'empreinte d'un joint élastique présentant, dans l'épaisseur de son extrémité terminale libre, une fente ou équivalent permettant le passage de l'aiguille lorsque le joint élastique est compressé en position connectée du connecteur.

En pratique, ce type de connecteur est utilisé à la fois pour effectuer des prélèvements de sang mais surtout pour l'injection de poches parentérales. Dans les deux cas, une fois l'opération achevée, on nettoie le cathéter avec du sérum physiologique ou de l'alcool.

Cependant, le connecteur qui vient d'être décrit, qui donne une certaine satisfaction peut encore être amélioré.

En effet, le retour du joint élastique à sa position initiale lors de la déconnexion du connecteur provoque une dépression au niveau de l'extrémité terminale de l'aiguille. Ce phénomène génère ainsi une aspiration du liquide présent dans le cathéter et entraine dans le même temps, une remontée de sang du patient à la base dudit cathéter, ce qui pose des problèmes d'hygiène évidents. En effet, le cathéter s'obstrue et crée un biofilm susceptible de s'infecter.

Pour résoudre ce problème, le document WO2008/052140 décrit un connecteur dont l'aiguille est munie de deux fentes longitudinales dans lesquelles, hors connexion, sont insérées des protubérances agencées sur la paroi interne du joint élastique. Dans la mesure où ces protubérances ne sont ménagées que sur partie seulement de la circonférence du joint, le liquide provenant des fentes lorsque celles-ci sont découvertes, s'écoule de part et d'autres des fentes, aussi bien dans le sens amont que dans le sens aval. En d'autres termes, au moment de la connexion, tout le volume disponible existant entre le fût de l'aiguille et la paroi interne du joint élastique est rempli de liquide. Compte tenu de la présence de boudins sur toute la hauteur du joint élastique, un volume résiduel de liquide entre les boudins de part et d'autres des orifices latéraux subsistera en position déconnectée. Ce liquide stagnant ne peut être purgé dans la mesure où les orifices sont bouchés après déconnexion. Il s'ensuit un risque de contamination évident.

Le document WO2006/013433A1 décrit un connecteur du même type si ce n'est que les orifices latéraux ne sont jamais obturés. De même que précédemment, du fait de la forme du joint élastique, un volume résiduel rempli de liquide subsiste entre la paroi du joint et celle de l'aiguille après déconnexion.

### EXPOSE DE L'INVENTION

Le but de l'invention est de remédier aux inconvénients précités en proposant un connecteur à usage médical n'entrainant aucune remontée indésirable de liquide lors de sa déconnexion et ne laissant subsister le moins possible de volume résiduel, avantageusement, aucun volume résiduel.

Pour ce faire, l'objectif de l'invention est de fournir un connecteur capable de générer, lors de sa déconnexion, une pression permettant de renvoyer un volume de liquide dans l'aiguille au moins égal au volume de liquide supplémentaire aspiré depuis le cathéter. Plus généralement, soit le volume renvoyé correspond au volume aspiré (on parle de valve neutre). Dans ce cas, le volume du cathéter est exclusivement rempli du liquide injecté. Soit le volume renvoyé est légèrement supérieur au volume aspiré (on parle de valve positive). Dans ce dernier cas, un faible volume du liquide injecté pénètre dans la veine du patient.

Pour atteindre cet objectif, il a été mis au point un connecteur à usage médical comprenant un raccord solidaire d'une chambre, le raccord étant muni en son centre d'une aiguille s'étendant dans ladite chambre et débouchant dans son extrémité terminale, laquelle présente une section apte à recevoir, par friction, un connecteur du type luer mâle pour la circulation d'un fluide, l'aiguille présentant une partie distale munie d'au moins un orifice latéral débouchant et étant contenue dans l'empreinte d'un joint élastique présentant, dans l'épaisseur de son extrémité libre, une fente ou équivalent, le joint élastique étant comprimé pendant la connexion pour découvrir la partie distale de l'aiguille et assurer le transfert de fluide, et relâché hors connexion.

L'aiguille présente, en dehors de sa partie distale, au moins un orifice supplémentaire et en ce que le joint élastique et l'aiguille sont conformés de telle sorte à ce que :
- lorsque le connecteur n'est pas connecté, le au moins un orifice supplémentaire est obturé par le joint élastique, et
- lorsque le connecteur est connecté, le au moins un orifice supplémentaire n'est pas obturé par le joint élastique.

Le connecteur se caractérise en ce que le joint élastique et l'aiguille sont conformés de telle sorte à ce que :
- lorsque le connecteur est connecté, le joint élastique à l'état comprimé forme avec l'aiguille, immédiatement en amont du niveau supérieur de l'orifice supplémentaire et sur portion seulement de la distance séparant ledit orifice de l'extrémité amont du joint, une cavité étanche destinée à recevoir par passage dans l'orifice supplémentaire une partie du fluide contenu dans l'aiguille,
- lorsque le joint élastique passe de l'état comprimé à l'état relâché :
   - la cavité contient encore du fluide lorsque le au moins un orifice latéral débouchant de l'aiguille est obturé par le joint élastique,
   - les parties du joint élastique, respectivement en aval et en amont de la cavité, de même que l'orifice supplémentaire sont conformés de sorte à ce que la partie aval du joint élastique obture le au moins un orifice latéral débouchant alors même que la cavité continue à déverser le fluide qu'elle contient dans l'aiguille.

Dans la description et dans les revendications, par l'expression « extrémité distale de l'aiguille », on désigne la partie de l'aiguille non recouverte par le joint élastique en position connectée du connecteur.

De même, le terme « connecté » signifie qu'il y a passage de liquide entre le luer mâle et l'aiguille.

Par ailleurs, l'expression « le joint élastique à l'état comprimé forme avec l'aiguille, immédiatement en amont du niveau supérieur de l'orifice supplémentaire et sur portion seulement de la distance séparant ledit orifice de l'extrémité amont du joint » exprime le fait que la cavité étanche ne s'étend pas, en amont du niveau supérieur de l'orifice supplémentaire, sur toute la longueur de l'aiguille mais sur partie seulement. Cela signifie que l'orifice est contenu dans la cavité étanche en position connectée et que le liquide ne peut s'écouler entre la paroi du joint élastique et le fut de l'aiguille de part et d'autre de la cavité ainsi délimitée.

Selon une caractéristique essentielle, la cavité est présente en position connectée du connecteur, peu importe qu'elle existe ou non avant connexion.

Lorsque la cavité n'existe pas avant connexion, celle-ci se forme immédiatement en amont du niveau supérieur dudit orifice supplémentaire, le volume de la cavité augmentant au fur et à mesure de la compression du joint élastique sur une portion seulement de la longueur de celui-ci. Lorsque la cavité existe avant connexion, celle-ci se déplace le long de l'aiguille, sous l'effet de la compression du joint élastique, en direction amont du dispositif.

Dès lors et en pratique, lors de la connexion, le joint élastique subit une compression sous l'effet de la force exercée par l'extrémité terminale du connecteur type luer mâle venant s'insérer dans l'extrémité terminale de la chambre. Cette compression a pour effet de libérer l'orifice supplémentaire et de former immédiatement en amont de son niveau supérieur (dans le sens du flux), un volume mort étanche entre la paroi interne du joint et la paroi de l'aiguille sur partie seulement de la distance séparant l'orifice supplémentaire de la base du joint ou de l'aiguille. Une partie du fluide circulant dans l'aiguille est aspiré par l'orifice supplémentaire dans la cavité ainsi formée. Au moment de la déconnexion, le joint retourne progressivement à sa position initiale par effet ressort. Ce relâchement entraine la remontée du fluide présent dans la cavité par raclage du bord inférieur de la cavité le long du fut de l'aiguille et la réintroduction de la totalité du volume dans le fut de l'aiguille via l'orifice supplémentaire. Selon une caractéristique essentielle, lorsque le joint élastique passe de l'état comprimé à l'état relâché, la cavité contient encore du fluide au moins jusqu'à ce que l'orifice débouchant de l'aiguille soit obturé par le joint élastique. Pour ce faire, les parties du joint élastique, respectivement en aval et en amont de la cavité, de même que l'orifice supplémentaire sont conformés de sorte à ce que la partie aval du joint élastique obture les orifices latéraux débouchant alors même que la cavité continue à déverser le fluide qu'elle contient dans l'aiguille. Dans ces conditions, on renvoie un certain volume de fluide dans l'aiguille destiné à venir compenser la dépression qui se produit au niveau de la partie terminale de l'aiguille au moment de la déconnexion et ainsi éviter toute remontée indésirable de fluide et/ou sang depuis le cathéter. En pratique, la partie du joint élastique en aval des orifices supplémentaires remonte le long de l'aiguille au moment de la déconnexion plus rapidement que la partie en amont. Notamment, la réintroduction du liquide dans le fut de l'aiguille par le biais de l'orifice supplémentaire, en fonction de son dimensionnement crée une résistance et freine nécessairement la remontée du joint. A l'issue de la manipulation, la cavité étanche ne contient pratiquement plus de liquide aux tolérances près de fabrication du joint elastique.

Selon l'invention, l'orifice supplémentaire est ménagé sur le fut de l'aiguille, avantageusement dans sa partie médiane.

Selon un premier mode de réalisation du connecteur (en position non connectée) :
- le joint élastique présente sur sa paroi interne un bourrelet périphérique apte à obturer le au moins un orifice supplémentaire,
- immédiatement en aval de l'orifice, sur partie au moins de la distance séparant ledit orifice de l'extrémité distale de l'aiguille, l'aiguille et la paroi interne du joint sont de forme complémentaire,
- immédiatement en amont de l'orifice supplémentaire, l'aiguille et la paroi interne du joint sont de forme non complémentaire.

En d'autres termes, la cavité se forme uniquement au moment de la connexion, par compression du joint élastique. En effet, la portion du joint en contact avec l'aiguille en aval de l'orifice subit un mouvement de translation amont. Tout en libérant l'orifice supplémentaire, un espace se crée entre la paroi du joint et le fut de l'aiguille qui est à ce niveau, de forme non complémentaire avec la forme de la paroi interne du joint élastique.

Dans un mode de réalisation préféré, l'aiguille et la paroi interne du joint sont de forme complémentaire sur partie seulement de la distance séparant ledit orifice de l'extrémité distale de l'aiguille.

Pour garantir la formation de la cavité en amont de l'orifice supplémentaire au moment de la compression du joint, la paroi de la partie du joint de forme complémentaire à celle de l'aiguille est plus rigide que celle du reste du joint. Dès lors, la partie du joint élastique en contact avec l'aiguille subit un mouvement de translation sous l'effet de la force exercée par le connecteur au moment de la connexion, entrainant la formation de la cavité en amont de l'orifice supplémentaire.

Avantageusement,
- la paroi interne du joint élastique présente une forme générale conique sur toute sa longueur,
- immédiatement en amont de l'orifice supplémentaire, l'aiguille présente une section externe cylindrique.

Dans un mode de réalisation particulier, immédiatement en amont de l'orifice supplémentaire, l'aiguille présente successivement un premier tronçon de section externe cylindrique puis un second tronçon conique de section externe croissante en direction de la base de l'aiguille.

Avantageusement, la section externe du second tronçon à sa naissance est supérieure à la section externe du premier tronçon de sorte à former un rebord périphérique d'appui et de fin de course du bourrelet périphérique apte à obturer le au moins un orifice supplémentaire en position comprimée du joint élastique, garantissant ainsi l'étanchéité de la chambre.

Dans un second mode de réalisation, le joint élastique présente une cavité préformée avant connexion.

Plus précisément et dans ce cas avantageusement (en position non connectée du connecteur) :
- le joint élastique présente sur sa paroi interne un premier bourrelet périphérique apte à obturer le au moins un orifice supplémentaire,
- immédiatement en aval de l'orifice supplémentaire, sur partie au moins de la distance séparant ledit orifice de l'extrémité distale de l'aiguille, l'aiguille et la paroi interne du joint sont de forme non complémentaire et délimitent une cavité étanche rigide,
- l'aiguille présente immédiatement en aval de l'orifice supplémentaire, un rebord périphérique de section identique ou presque à celle de la cavité.

Avantageusement, immédiatement en aval du bourrelet périphérique, la paroi interne du joint présente un tronçon de forme tubulaire délimité en aval par un second bourrelet périphérique maintenu en contact serré avec l'aiguille en position relâchée du joint.

Dans ce cas, au moment de la connexion, la cavité préformée effectue un mouvement de translation en amont par rapport à l'aiguille. En découvrant l'orifice, la cavité se remplit de fluide. Du fait de la présence du rebord périphérique, la paroi de la cavité est en contact serré avec ledit rebord de sorte que la cavité formée en dessous du niveau supérieur de l'orifice supplémentaire se remplit de liquide et reste étanche. Le rebord périphérique sert d'appui et de fin de course au second bourrelet périphérique en position comprimée du joint élastique. Lorsque le joint reprend sa position initiale, la cavité se vide en subissant le même mouvement de translation mais en direction aval et ce, sans que le liquide ne circule entre la paroi élastique et le fut de l'aiguille au-delà du rebord périphérique. De même que précédemment, dans ces conditions aucun liquide ne subsiste dans la cavité après déconnexion

Selon une autre caractéristique, la paroi du tronçon de forme tubulaire est plus rigide que celle du reste du joint. Avantageusement, le tronçon du joint de forme tubulaire est rainuré. Comme déjà dit, dans tous les modes de réalisation, pour garantir que la cavité continue à déverser du fluide dans l'aiguille lorsque l'orifice débouchant de l'aiguille est obturé par le joint élastique, les parties du joint élastique, respectivement en aval et en amont de la cavité, de même que l'orifice supplémentaire sont conformés de sorte à ce que, lorsque le joint passe de l'état comprimé à l'état relâché, la partie aval du joint élastique remonte plus rapidement que la partie amont. Pour ce faire, il est nécessaire de trouver un compromis entre l'élasticité de la partie du joint en amont de la cavité et celle située en aval de ladite cavité, le tout en considérant la taille de l'orifice qui constitue une résistance supplémentaire lorsque le fluide repart dans l'aiguille.

Dans un mode de réalisation préféré, la paroi du joint en amont et en aval de la cavité se présente sous la forme d'une succession de bourrelets conférant aux parties du joint en aval et en amont de la cavité une rigidité identique.

Le nombre d'orifice supplémentaire n'est pas limité à la condition que leur positionnement soit compatible avec le fonctionnement du connecteur.

En pratique, les orifices supplémentaires sont au nombre de 2 et positionnés en regard l'un de l'autre.

### BREVE DESCRIPTION DES DESSINS

L'invention sera bien comprise et d'autres caractéristiques et avantages de l'invention ressortiront clairement de la description qui en est faite ci-après, à titre indicatif et nullement limitatif, en référence aux figures annexées, dans lesquelles :
- la figure 1 est une représentation schématique en perspective d'un connecteur à usage médical selon l'invention.
- les figures 2 à 10 sont des représentations schématiques en coupe longitudinale du connecteur selon l'invention selon un premier mode de réalisation.
- les figures 11 à 15 sont des représentations schématiques en coupe longitudinale du connecteur selon l'invention selon un second mode de réalisation.

### EXPOSE DETAILLE DE L'INVENTION

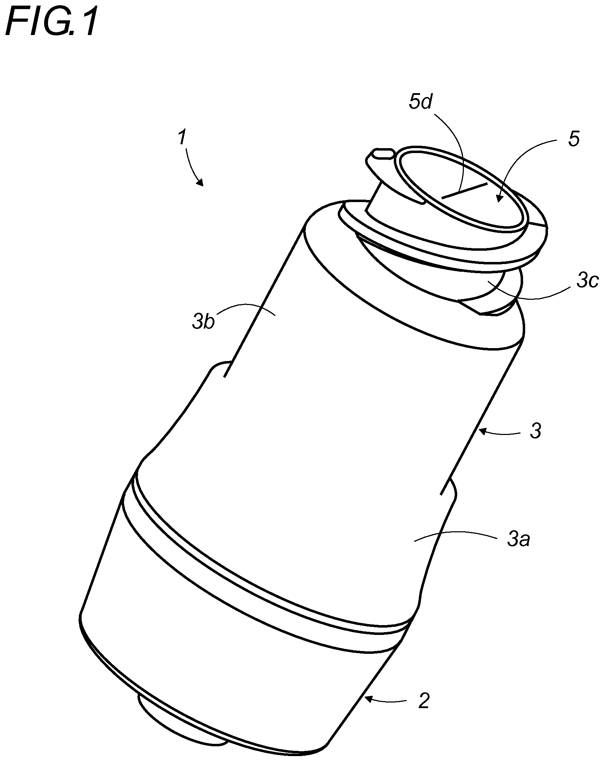

En référence notamment à la figure 1 représentant un connecteur (1) à usage médical selon l'invention, celui-ci comprend un raccord (2) solidaire d'une chambre (3) ainsi qu'un assemblage bague (4) / joint élastique (5). Le raccord (2) se présente sous la forme d'une pièce composite associant le corps du raccord (2) proprement dit et une aiguille (6). La chambre (3) comprend un compartiment proximal (3a), un compartiment central (3b) et un compartiment distal (3c).

Le compartiment distal (3c) de la chambre (3) présente une section apte à recevoir, par friction, un connecteur (7) de type luer mâle. Le compartiment proximal (3a) de la chambre (3) coopère avec le raccord (2), destiné à recevoir un cathéter reliant le patient et dans lequel circule un fluide.

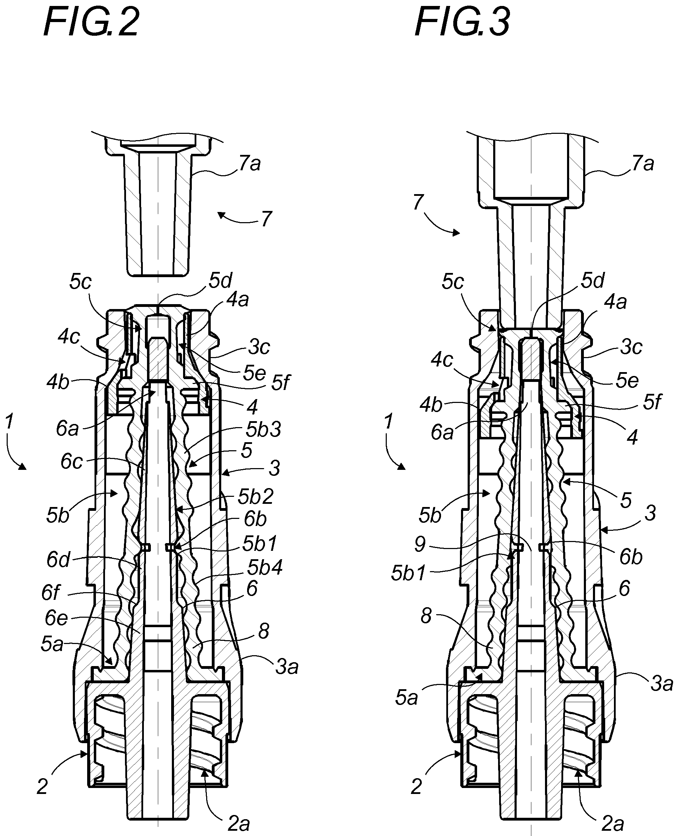

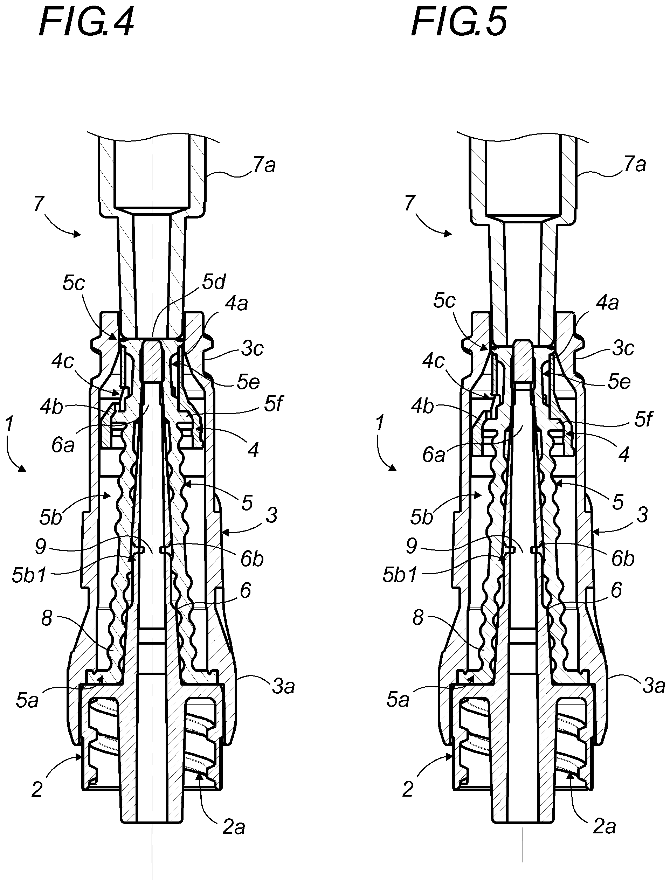

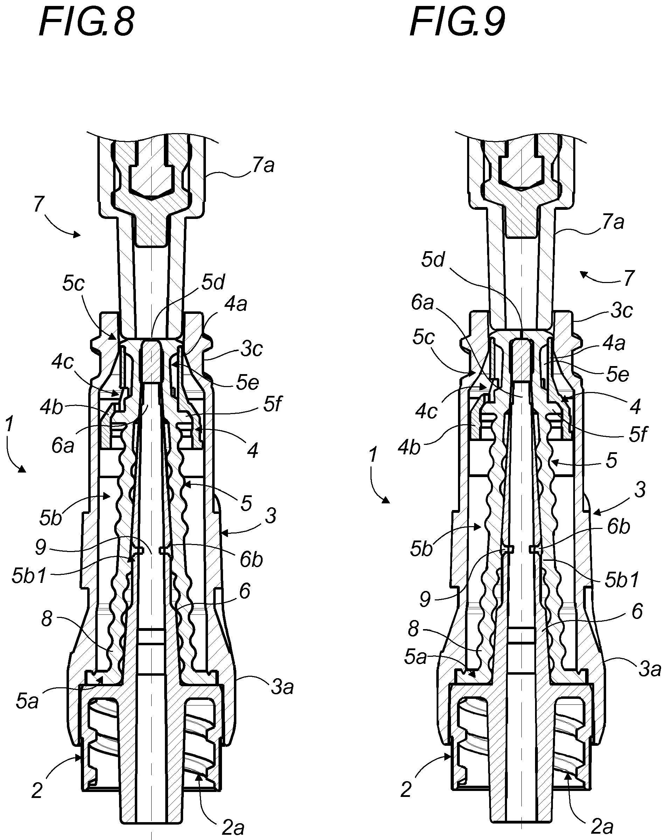

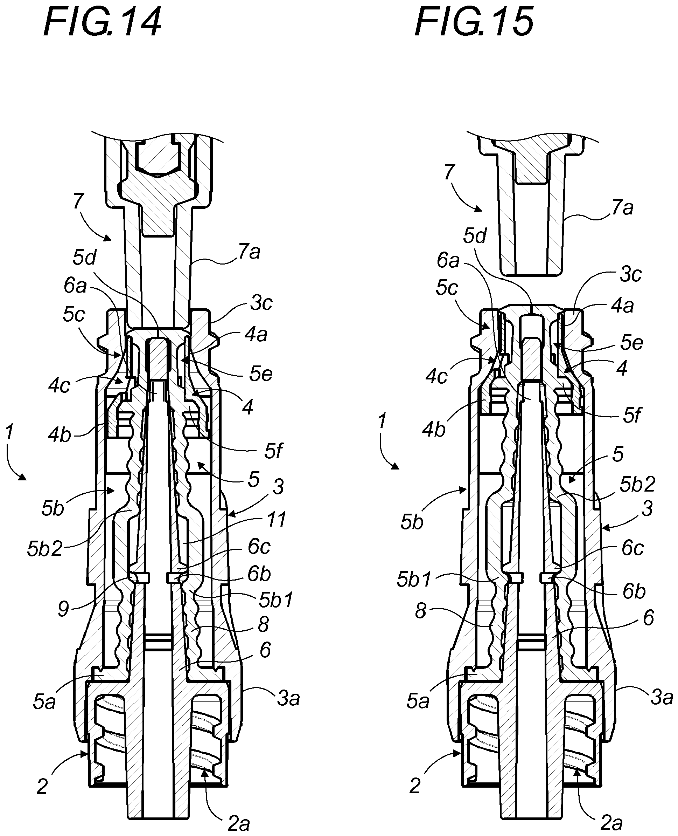

Comme le montrent les figures 2 à 15, le corps du raccord (2) comprend un évidement (2a), muni sur sa face interne d'un pas de vis destiné à coopérer avec un pas de vis correspondant d'un cathéter ou d'un autre connecteur luer femelle dans laquelle peut circuler un fluide. Le corps du raccord (2) est muni en son centre d'une aiguille (6) s'étendant depuis le compartiment proximal (3a) jusqu'au compartiment distal (3c).

L'aiguille (6) proprement dite est munie, à proximité de son extrémité libre, elle-même obturée, de deux orifices supérieurs latéraux (6a) par lesquels s'écoule le fluide lorsque sa partie distale est découverte, c'est-à-dire en position connectée. L'aiguille (6) est en effet gainée sur toute sa longueur dans l'empreinte d'un joint élastique (5) dont la partie terminale est cerclée par une bague (4). L'extrémité distale au moins de l'aiguille (6) présente une forme sensiblement conique de manière à favoriser son dégagement sous l'effet de la poussée exercée par le connecteur (7) type luer mâle sur l'ensemble bague (4)/joint élastique (5).

L'aiguille (6) comprend selon l'invention, deux orifices supplémentaires inférieurs latéraux (6b), ménagés dans sa partie médiane. Ils sont diamétralement opposés l'un à l'autre.

Dans le mode de réalisation objet des figures 2 à 10, l'aiguille présente en aval des orifices (6b) une section conique (6c). En amont des orifices (6b), l'aiguille présente successivement un premier tronçon (6d) de section externe cylindrique puis un second tronçon (6e) de section externe conique croissante en direction de la base de l'aiguille. A sa naissance, le second tronçon présente un diamètre supérieur à celui du premier tronçon de sorte à former un rebord périphérique (6f).

Le joint élastique (5) est réalisé en silicone et se présente sous la forme d'un tube de section externe de forme générale conique, destiné à s'étendre dans la chambre (3) depuis le corps du raccord (2) jusqu'à l'extrémité libre du compartiment terminal (3c) de la chambre (3). Le joint (5) comprend une partie formant base (5a), une partie médiane (5b) et une partie terminale (5c).

La partie formant base (5a) du joint élastique (5) est destinée à reposer sur le corps du raccord (2). En position non comprimée, c'est-à-dire en position relâchée, ni la partie formant base (5a) du joint élastique (5), ni la partie médiane (5b) ne sont en contact avec les parois de la chambre (3).

La paroi interne de la partie médiane (5b) du joint élastique (5) comprend un bourrelet périphérique (5b1) positionné de sorte à obturer les orifices latéraux inférieurs (6b) de l'aiguille en position relâchée du joint, c'est-à-dire hors connexion.

Directement en aval de ce bourrelet, la paroi interne du joint présente une forme conique (5b2) complémentaire de celle de l'aiguille, en contact avec celle-ci. La face externe du joint à ce niveau est rectiligne et plus rigide que le reste du joint. Le caractère rigide peut être renforcé en ménageant des rainures longitudinales sur la portion de joint correspondante. En aval de cette zone rectiligne 5b2, la paroi du joint élastique 5b3 jusqu'à son extrémité distale est constituée d'une succession de bourrelets. De même, dans sa partie 5b4 située en amont du bourrelet périphérique (5b1), la paroi du joint est constituée d'une succession de bourrelets jusqu'à son extrémité amont.

La cinématique du joint élastique au moment de la connexion et de la déconnexion est la suivante.

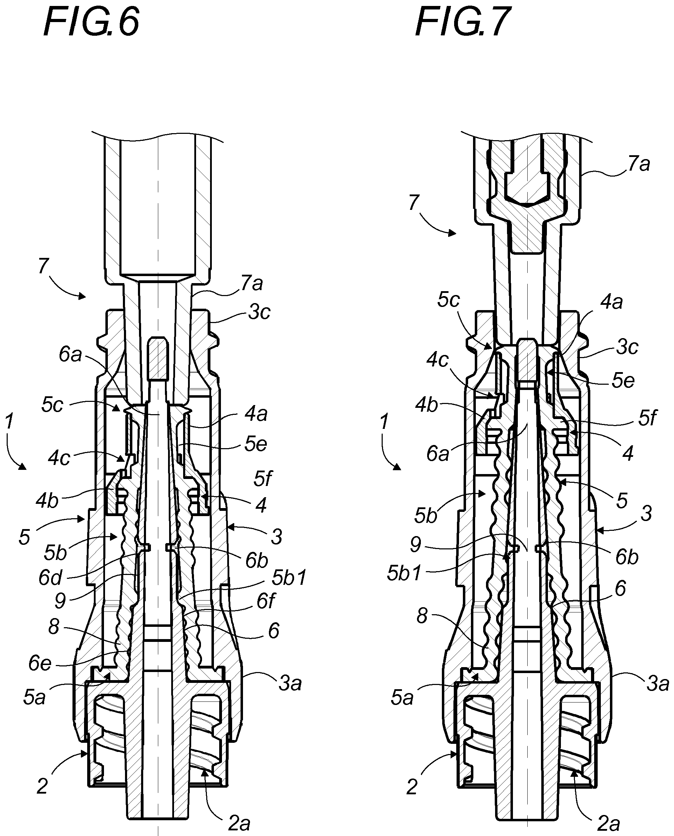

Comme le montrent les figures 3 à 5, sous l'effet de la pression exercée par le luer mâle sur le joint élastique, la partie en aval du bourrelet inférieur (5b1) se comprime plus rapidement que la partie en amont. Ainsi, le bourrelet inférieur (5b1) subit un mouvement de translation sur une distance très courte, permettant de dégager les orifices supplémentaires (6b). Dans le même temps, la partie en aval subit un mouvement de translation sur une distance supérieure, permettant ainsi de dégager progressivement l'extrémité distale de l'aiguille. Comme le montre la figure 6, le mouvement de translation que subit ensuite le bourrelet inférieur (5b1) entraine son déplacement jusqu'au rebord périphérique (6f) de l'aiguille (6). Un volume mort étanche (9), délimité par la paroi cylindrique de l'aiguille (6d), la paroi interne (5b2) de la partie médiane (5b) du joint élastique (5) et ledit bourrelet (5b1) se forme simultanément et se remplit du liquide circulant dans l'aiguille. Le joint a terminé sa course lorsque le bourrelet (5b1) vient en appui sur rebord périphérique (6f). Dans le même temps, la partie du joint élastique en aval des orifices supplémentaires est suffisamment comprimée pour découvrir les orifices latéraux supérieurs (6a). Le liquide peut alors circuler depuis le connecteur luer mâle dans l'aiguille du connecteur de l'invention.

Lorsque le joint élastique (5) retourne dans une position relâchée, le bourrelet inférieur (5b1) remonte progressivement et pousse le liquide présent dans le volume mort étanche (9) à l'intérieur de l'aiguille (6) par les orifices latéraux inférieurs (6b). La cinématique du joint élastique (figures 7-9) est la même que celle constatée au moment de la compression (figures 3-5) mais en sens inverse. Comme le montre la figure (10) le bourrelet inférieur (5b1) retourne dans sa position d'origine, c'est-à-dire en position d'obturation desdits orifices latéraux inférieurs (6b) de l'aiguille (6). Selon l'invention pour assurer cette cinétique spécifique, le volume de la cavité, les caractéristiques élastiques des parties 5b3 et 5b4 du joint de même que la taille de l'orifice supplémentaire sont calculés pour que la partie 5b3 du joint en aval de la cavité remonte plus vite que la partie en amont de la cavité. Dès lors, la cavité ne se vide pas complètement avant que les orifices débouchant de l'aiguille ne soit complètement obturés par le joint en position déconnectée. La réinjection du contenu de la cavité dans le fut de l'aiguille lorsque le joint revient à sa position de repos permet ainsi de compenser la remontée de liquide générée par la déconnexion, en particulier de sang, dans la tubulure.

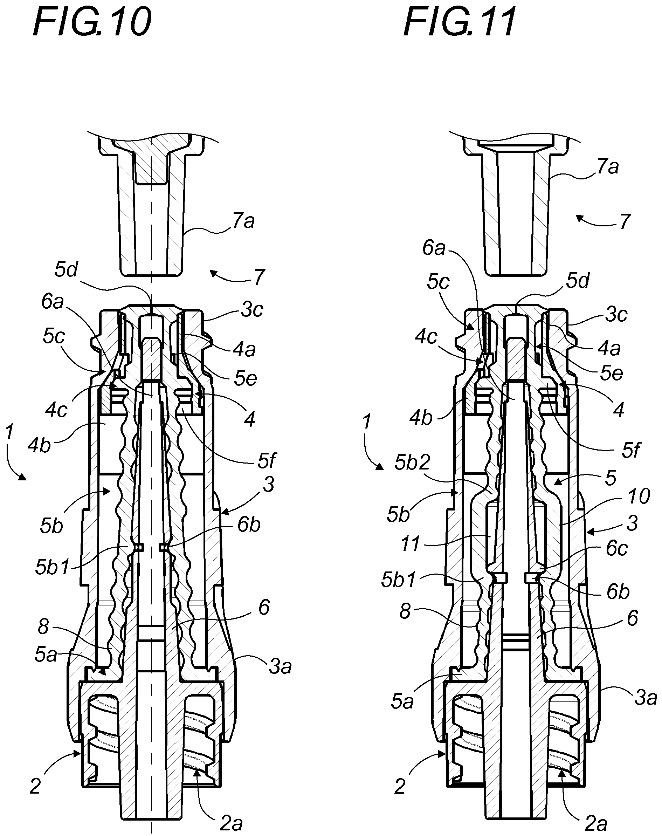

Dans une seconde forme de réalisation de l'invention, représentée aux figures 11 à 15, le joint élastique comprend une cavité préformée.

Dans ce mode de réalisation, le joint élastique présente encore sur sa paroi interne, un bourrelet périphérique (5b1) désigné dans ce cas «premier bourrelet » apte à obturer les deux orifices supplémentaires (6b).

Immédiatement en aval du premier bourrelet périphérique (5b1), la paroi interne du joint présente un tronçon de forme tubulaire (10) délimité en aval par un second bourrelet périphérique (5b2) maintenu en contact serré avec l'aiguille. L'ensemble forme ainsi une cavité (11) dont la paroi (10) est plus rigide que celle du reste du joint.

Toujours dans ce second mode de réalisation, l'aiguille présente directement en aval de l'orifice supplémentaire, un rebord périphérique (6c) d'appui et de fin de course du second bourrelet périphérique (5b2) en position comprimée du joint élastique. Ce bourrelet (6c) permet de conférer au volume mort (9) une étanchéité optimale en position connectée. Il fait également office de butée pour le bourrelet inférieur (5b1) en position relâchée du joint élastique (5). De la sorte, le liquide ne peut circuler en aval du rebord périphérique (6c) en direction aval, entre la paroi élastique et le fut de l'aiguille.

De même que précédemment, le volume de la cavité, les caractéristiques élastiques des parties du joint en amont et en aval de la cavité de même que la taille de l'orifice supplémentaire sont calculés pour que la partie du joint en aval de la cavité remonte plus vite que la partie en amont de la cavité.

En pratique, lors de la compression du joint élastique (5), c'est-à-dire au moment de la connexion avec un connecteur (7) type luer mâle, la cavité (11) subit un mouvement de translation en direction amont du connecteur. Le bourrelet inférieur (5b1) découvre ainsi les orifices (6c), de sorte que la cavité se remplit progressivement du fluide circulant dans le fut de l'aiguille. La cavité termine sa course lorsque le second bourrelet périphérique (5b2) vient en appui sur le rebord périphérique de l'aiguille (6c).

Lorsque le joint élastique (5) retourne dans une position initiale par effet ressort, le bourrelet inférieur (5b1) remonte et pousse le liquide, par un phénomène de raclage, à l'intérieur de l'aiguille (6) via les orifices latéraux inférieurs (6b), et ce, jusqu'à que ledit bourrelet inférieur (5b1) retourne dans sa position d'origine d'obturation des orifices latéraux inférieurs (6b) de l'aiguille (6). Avant que les orifices latéraux inférieurs ne soit obturés, la partie du joint en aval de la cavité a déjà obturé les orifices débouchant de l'aiguille. Comme déjà mentionné, le volume de liquide est confiné dans la partie de la cavité située en amont de l'orifice supplémentaire et ne peut circuler au-delà du rebord périphérique (6b) que ce soit lors de la connexion ou de la deconnexion.

Le volume de fluide ainsi réintroduit dans le fut de l'aiguille crée une pression permettant de compenser la dépression engendrée par la déconnexion au niveau de la partie terminale de l'aiguille (6) et ainsi éviter tout risque de remontée indésirable de fluide.

Dans le connecteur (1) selon l'invention, quel que soit le mode de réalisation, la partie terminale (5c) du joint élastique (5) est en outre munie d'une fente (5d), permettant le passage de l'aiguille (6), lorsque le joint élastique (5) se trouve dans une position dite « comprimée ». La partie terminale (5c) du joint élastique (5) comprend en outre un évidement (5e) en amont de l'extrémité de l'aiguille (6) en position non comprimée du joint élastique (5). La partie terminale (5c) présente également une collerette (5f), qui permet le guidage et le centrage de ladite partie terminale (5c) du joint élastique (5) dans le compartiment central (3b) de la chambre (3). Pour tenir compte de la forme et des dimensions du compartiment central (3b) de la chambre (3), cette collerette (5f) est également cerclée par la bague (4).

La bague (4) est positionnée à l'extrémité du joint élastique (5) et présente deux tronçons de sections différentes, respectivement un premier tronçon cylindrique (4a) cerclant la partie terminale (5c) du joint élastique (5) sur une longueur correspondant à la longueur du compartiment terminal (3c) et de section sensiblement égale à la section dudit compartiment terminal (3c) et un second tronçon cylindrique (4b), de section supérieure sensiblement égale à la section correspondante du compartiment central (3b) et recouvrant la collerette (5f) du joint élastique (5). La bague (4) est réalisée en un matériau rigide ou semi-rigide, éventuellement en bi-matière avec le joint élastique (5), ou séparément, bague (4) et joint (5) étant rendus solidaires l'un de l'autre notamment par collage ou simple apposition. La section externe du joint (5) est sensiblement égale à la section interne de la bague (4) sur leur zone de recouvrement.

Telle que représenté aux figures 2 à 15, la partie terminale et plus particulièrement le premier tronçon cylindrique (4a) de la bague (4) présente deux ajourements (4c) destinés à favoriser le refoulement de la matière plastique au moment du passage de l'aiguille (6) sous l'effet de la poussée créée par la mise en place du connecteur (7) type luer mâle.

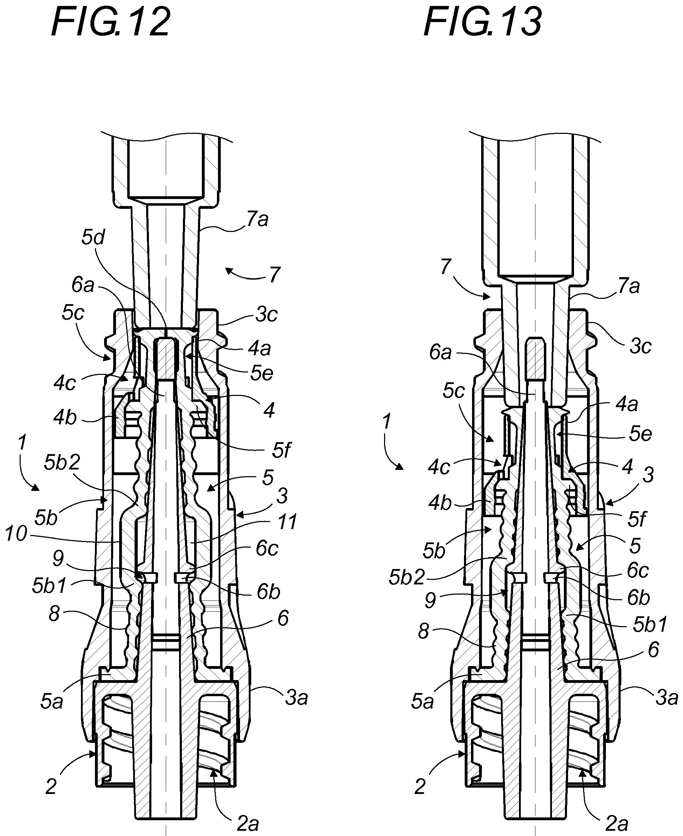

Le compartiment central (3b) et le compartiment terminal (3c) sont reliés par un épaulement servant de butée à la bague (4) cerclant l'extrémité terminale (5c) du joint élastique (5) en position de repos, c'est-à-dire en position de relâchement du joint.
La position connectée est plus précisément représentée aux figure 6 et 13 par la mise en place d'un connecteur (7) type luer mâle. Ce connecteur (7) est en outre muni d'un cône luer (7a), destiné à être insérée dans la lumière du compartiment terminal (3c) de la chambre (3). En pratique, le cône (7a) vient prendre appui par son extrémité sur l'extrémité libre de l'ensemble bague (4)/joint élastique (5). Cet appui provoque le déplacement du joint élastique (5) le long du fût de l'aiguille (6) en direction du raccord (2), puis le passage de la pointe de l'aiguille (6) au travers de la fente (5d), facilité par les évidements (5e), et enfin la libération des orifices supérieurs latéraux (6a) et autorisant le passage du fluide.

En position connectée, l'extrémité distale de l'aiguille (6) est donc dans sa totalité, contenue dans le canal intérieur du cône (7a), permettant ainsi la transmission du fluide de connecteur (1) à connecteur (7). Le mouvement de la bague (4) pendant cette opération, est un mouvement axial homogène et uniforme de par le contact permanent des parois de la section de la bague (4) avec le compartiment central (3b) de la chambre (3) tout au long du mouvement, moyennant un minimum de friction.

Comme il ressort de ce qui précède, l'invention fournit un connecteur (1) à usage médical donnant entière satisfaction d'utilisation. On note en particulier la compensation de la remontée indésirable de fluide liée à la déconnexion du connecteur (1).

## Revendications

1. Connecteur (1) à usage médical comprenant un raccord (2) solidaire d'une chambre (3), le raccord (2) étant muni en son centre d'une aiguille (6) s'étendant dans ladite chambre (3) et débouchant dans son extrémité terminale, laquelle présente une section apte à recevoir, par friction, un connecteur (7) type luer mâle pour la circulation d'un fluide, l'aiguille (6) présentant une partie distale munie d'au moins un orifice latéral débouchant (6a) et étant contenue dans l'empreinte (5g) d'un joint élastique (5) présentant, dans l'épaisseur de son extrémité libre, une fente (5d) ou équivalent, le joint élastique étant comprimé pendant la connexion pour découvrir la partie distale de l'aiguille et assurer le transfert de fluide, et relâché hors connexion, l'aiguille présentant, en dehors de sa partie distale, au moins un orifice supplémentaire (6b), le joint élastique et l'aiguille (6) étant conformés de telle sorte à ce que :
- lorsque le connecteur n'est pas connecté, le au moins un orifice supplémentaire (6b) est obturé par le joint élastique (5), et
- lorsque le connecteur est connecté, le au moins un orifice supplémentaire (6b) n'est pas obturé par le joint élastique (5) ;
***caractérisé en ce que*** le joint élastique et l'aiguille (6) sont également conformés de telle sorte à ce que :
- lorsque le connecteur (1) est connecté, le joint élastique (5) à l'état comprimé forme avec l'aiguille (6), immédiatement en amont du niveau supérieur de l'orifice supplémentaire (6b) et sur portion seulement de la distance séparant ledit orifice de l'extrémité amont du joint, une cavité étanche (9) destinée à recevoir par passage dans l'orifice supplémentaire (6b) une partie du fluide contenu dans l'aiguille (6), et
- lorsque le joint élastique (5) passe de l'état comprimé à l'état relâché :
• la cavité (9) contient encore du fluide lorsque le au moins un orifice latéral débouchant (6a) de l'aiguille (6) est obturé par le joint élastique (5),
• les parties du joint élastique (5), respectivement en aval et en amont de la cavité (9), de même que l'orifice supplémentaire (6b) sont conformés de sorte à ce que la partie aval du joint élastique (5) obture le au moins un orifice latéral débouchant (6a) alors même que la cavité (9) continue à déverser le fluide qu'elle contient dans l'aiguille (6).

2. Connecteur selon la revendication 1, **caractérisé en ce que** l'orifice supplémentaire (6b) est ménagé dans la partie médiane de l'aiguille (6).

3. Connecteur selon la revendication 1 ou 2, **caractérisé en ce que** :
- le joint élastique (5) présente sur sa paroi interne un bourrelet périphérique (5b1) apte à obturer le au moins un orifice supplémentaire (6b),
- immédiatement en aval de l'orifice (6b), sur partie au moins de la distance séparant ledit orifice de l'extrémité distale de l'aiguille (6), l'aiguille (6) et la paroi interne du joint (5) sont de forme complémentaire,
- immédiatement en amont de l'orifice supplémentaire, l'aiguille (6) et la paroi interne du joint (5) sont de forme non complémentaire.

4. Connecteur selon la revendication 3, **caractérisé en ce que** l'aiguille (6) et la paroi interne du joint (5) sont de forme complémentaire sur partie seulement de la distance séparant ledit orifice de l'extrémité distale de l'aiguille.

5. Connecteur selon l'une des revendications 3 ou 4, **caractérisé en ce que** la paroi de la partie du joint de forme complémentaire à celle de l'aiguille (6) est plus rigide que celle du reste du joint.

6. Connecteur selon l'une des revendications 3 à 5, **caractérisé en ce que** :
- la paroi interne du joint élastique (5) présente une forme générale conique sur toute sa longueur,
- immédiatement en amont de l'orifice supplémentaire (6b), l'aiguille (6) présente une section externe cylindrique.

7. Connecteur selon la revendication 6, **caractérisé en ce qu'**immédiatement en amont de l'orifice supplémentaire (6b), l'aiguille (6) présente successivement un premier tronçon de section externe cylindrique (6d) puis un second tronçon conique de section externe croissante (6e) en direction de la base de l'aiguille.

8. Connecteur selon la revendication 1 ou 2, **caractérisé en ce que** :
- le joint élastique (5) présente sur sa paroi interne un premier bourrelet périphérique (5b1) apte à obturer le au moins un orifice supplémentaire (6b),
- immédiatement en aval de l'orifice supplémentaire (6b), sur partie au moins de la distance séparant ledit orifice de l'extrémité distale de l'aiguille (6), l'aiguille et la paroi interne du joint (5) sont de forme non complémentaire et délimitent une cavité étanche rigide (9),
- l'aiguille présente immédiatement en aval de l'orifice supplémentaire, un rebord périphérique de section sensiblement identique à celle de la cavité.

9. Connecteur selon la revendication 8, **caractérisée en ce que**, immédiatement en aval du bourrelet périphérique (5b1), la paroi interne du joint (5) présente un tronçon de forme tubulaire délimité en aval par un second bourrelet périphérique (5b) maintenu en contact serré avec l'aiguille (6).

10. Connecteur selon l'une des revendications 8 ou 9, **caractérisé en ce que** la paroi du tronçon de forme tubulaire est plus rigide que celle du reste du joint.

11. Connecteur selon l'une des revendications précédentes, **caractérisé en ce que** la paroi du joint en amont et en aval de la cavité se présente sous la forme d'une succession de bourrelets conférant aux parties du joint en aval et en amont de la cavité une rigidité identique.

12. Connecteur selon l'une des revendications précédentes, **caractérisé en ce que** les orifices supplémentaires sont au nombre de 2 et positionnés en regard l'un de l'autre.

## Patentansprüche

1. Medizinscher Verbinder (1) mit einem Anschlussstück (2), das mit einer Kammer (3) fest verbunden ist, wobei das Anschlussstück (2) dabei in seinem Zentrum über eine Nadel (6) verfügt, die in diese Kammer (3) ragt und in ihrem terminalen Endstück endet, das einen Querschnitt aufweist, in dem reibschlüssig ein Verbinder (7), vom Typ männlicher Luer Lock-Konnektor, zur Zirkulation einer Flüssigkeit aufgenommen werden kann, wobei die Nadel (6) einen distalen Teil aufweist, der mit mindestens einer seitlichen Öffnung (6a) versehen ist und dabei in der Aussparung (5g) einer elastischen Dichtung (5) enthalten ist, die in der Dicke ihres freien Endstückes einen Spalt (5d) oder Vergleichbares aufweist, wobei die elastische Dichtung dabei während der Verbindung zusammengepresst wird, so dass der distale Teil der Nadel freigelegt ist und die Flüssigkeit durchströmen kann und wobei sie entspannt ist, wenn keine Verbindung besteht, wobei die Nadel außerhalb ihres distalen Teils mindestens eine zusätzliche Öffnung (6b) aufweist, wobei die elastische Dichtung und die Nadel (6) so geformt sind, dass:
- wenn der Verbinder nicht verbunden ist, die mindestens eine zusätzliche Öffnung (6b) von der elastischen Dichtung (5) verschlossen wird, und
- wenn der Verbinder verbunden ist, die mindestens eine zusätzliche Öffnung (6b) von der elastischen Dichtung (5) nicht verschlossen wird, und
**dadurch gekennzeichnet, dass** die elastische Dichtung und die Nadel (6) ebenfalls so geformt sind, dass:
- wenn der Verbinder (1) verbunden ist, die elastische Dichtung (5) im komprimierten Zustand mit der Nadel (6), direkt oberhalb des oberen Niveau der zusätzlichen Öffnung (6b) und nur auf dem Abschnitt des Abstandes, der diese Öffnung vom Endstück oberhalb der Dichtung trennt, eine dichte Vertiefung (9) bildet, die dazu bestimmt ist, durch Durchfluss durch die zusätzliche Öffnung (6b) einen Teil der in der Nadel (6) enthaltenen Flüssigkeit aufzunehmen und
- wenn die elastische Dichtung (5) aus dem komprimierten Zustand in den entspannten Zustand übergeht:
• enthält die Vertiefung (9) weiterhin Flüssigkeit, wenn die mindestens eine einmündende seitliche Öffnung (6a) der Nadel (6) von der elastischen Dichtung (5) verschlossen ist,
• sind die Teile der elastischen Dichtung (5), jeweils unterhalb und oberhalb der Vertiefung (9), ebenso wie die zusätzliche Öffnung (6b) so ausgebildet, dass der unterhalb liegende Teil der elastischen Dichtung (5) die mindestens eine einmündende seitliche Öffnung (6a) verschließt, obwohl die Vertiefung (9) weiterhin die Flüssigkeit abgibt, die sie in der Nadel (6) enthält.

2. Verbinder nach Anspruch 1, **dadurch gekennzeichnet, dass** die zusätzliche Öffnung (6b) im medianen Teil der Nadel (6) ausgespart ist.

3. Verbinder nach Anspruch 1 oder 2, **dadurch gekennzeichnet, dass**:
- die elastische Dichtung (5) auf der Innenwand einen umlaufenden Wulst (5b1) aufweist, der in der Lage ist, die mindestens eine zusätzliche Öffnung (6b) zu verschließen,
- unmittelbar hinter der Öffnung (6b), zumindest über einen Teil des Abstandes hinweg, der diese Öffnung vom distalen Endstück der Nadel (6) trennt, die Nadel (6) und die Innenwand der Dichtung (5) eine komplementäre Form haben,
- unmittelbar oberhalb der zusätzlichen Öffnung die Nadel (6) und die Innenwand der Dichtung (5) keine komplementäre Form haben.

4. Verbinder nach Anspruch 3, **dadurch gekennzeichnet, dass** die Nadel (6) und die Innenwand der Dichtung (5) eine komplementäre Form nur in dem Teil des Abstandes haben, der diese Öffnung vom distalen Endstück der Nadel trennt.

5. Verbinder nach einem der Ansprüche 3 oder 4, **dadurch gekennzeichnet, dass** die Wand des Teils der Dichtung mit einer Form, die zu der der Nadel (6) komplementär ist, steifer ist, als die der übrigen Dichtung.

6. Verbinder nach einem der Ansprüche 3 bis 5, **dadurch gekennzeichnet, dass**:
- die Innenwand der elastischen Dichtung (5) über ihre gesamte Länge hinweg eine im Allgemeinen konische Form aufweist,
- unmittelbar oberhalb der zusätzlichen Öffnung (6b) die Nadel (6) einen zylindrischen Außenquerschnitt hat.

7. Verbinder nach Anspruch 6, **dadurch gekennzeichnet, dass** unmittelbar hinter der zusätzlichen Öffnung (6b) die Nadel (6) nacheinander ein erstes Teilstück mit einem zylindrischen Außenquerschnitt (6d), dann ein zweites, konisches Teilstück mit zunehmendem Außenquerschnitt (6e) in Richtung der Nadelbasis aufweist.

8. Verbinder nach Anspruch 1 oder 2, **dadurch gekennzeichnet, dass**:
- die elastische Dichtung (5) auf der Innenwand einen ersten umlaufenden Wulst (5b1) aufweist, der in der Lage ist, die mindestens eine zusätzliche Öffnung (6b) zu verschließen,
- unmittelbar unterhalb der zusätzlichen Öffnung (6b), zumindest über einen Teil des Abstandes hinweg, der diese Öffnung vom distalen Endstück der Nadel (6) trennt, die Nadel und die Innenwand der Dichtung (5) eine nicht komplementäre Form haben und eine starre, dichte Vertiefung (9) begrenzen,
- die Nadel unmittelbar unterhalb der zusätzlichen Öffnung einen umlaufenden Rand aufweist, dessen Querschnitt im Wesentlichen mit dem der Vertiefung übereinstimmt.

9. Verbinder nach Anspruch 8, **dadurch gekennzeichnet, dass** unmittelbar unterhalb des umlaufenden Wulstes (5b1), die Innenwand der Dichtung (5) einen rohrförmigen Abschnitt aufweist, der unterhalb von einem zweiten umlaufenden Wulst (5b) begrenzt ist, der engen Kontakt mit der Nadel (6) hat.

10. Verbinder nach einem der Ansprüche 8 oder 9, **dadurch gekennzeichnet, dass** die Wand des rohrförmigen Teilstücks starrer ist als die der übrigen Dichtung.

11. Verbinder nach einem der vorangehenden Ansprüche, **dadurch gekennzeichnet, dass** die Wand der Dichtung oberhalb und unterhalb der Vertiefung die Form aufeinanderfolgender Wülste hat, die den Teilen der Dichtung unterhalb und oberhalb der Vertiefung eine identische Steifigkeit verleiht.

12. Verbinder nach einem der vorangehenden Ansprüche, **dadurch gekennzeichnet, dass** er 2 zusätzliche Öffnungen enthält, die einander gegenüberliegend angeordnet sind.

## Claims

1. Medical connector (1) comprising a joint (2) fixed to a chamber (3), the joint (2) being provided at its centre with a needle (6) extending into said chamber (3) and opening into its terminal end, which has a section suitable for receiving, by friction, a male luer connector (7) for the circulation of a fluid; the needle (6) has a distal portion with at least one lateral hole (6a) opening into and contained in a cavity (5g) of a resilient seal (5) having, in the thickness of its free end, a slit (5d) or a similar opening, the resilient seal being compressed when connected to uncover the distal portion of the needle and ensure the transfer of fluid, and released when disconnected, the needle having, apart from its distal portion, at least one additional hole (6b), the resilient seal and the needle (6) being shaped so that:
- when the connector is not connected, at least one additional hole (6b) is closed by the resilient seal (5), and
- when the connector is connected, at least one additional hole (6b) is not closed by the resilient seal (5);
***characterised in that*** the resilient seal and the needle (6) are also shaped so that:
- when the connector (1) is connected, the resilient seal (5) in the compressed state forms with the needle (6), immediately upstream of the upper level of the additional hole (6b) and only on the portion of the distance separating the said hole of the upstream end from the seal, a sealed cavity (9) for receiving by way of passage in the additional hole (6b) a part of the fluid contained in the needle (6), and
- when the resilient seal (5) goes from the compressed state to the released state:
• the cavity (9) still contains fluid when the at least one lateral hole (6b) opening into the needle (6) is sealed by the resilient seal (5),
• the portions of the resilient seal (5), downstream and upstream of the cavity (9) respectively, as well as the additional hole (6b) are shaped so that the downstream portion of the resilient seal (5) closes at least one lateral hole (6a) while the same cavity (9) continues to discharge the fluid contained therein into the needle (6).

2. Connector according to claim 1, **characterised in that** the additional hole (6b) is formed in the middle portion of the needle (6).

3. Connector according to one of claims 1 or 2, **characterised in that**:
- the resilient seal (5) has on its inner wall a peripheral pad (5b 1) adapted to close at least one additional hole (6b),
- immediately downstream of the hole (6b), on at least part of the distance separating said hole from the distal end of the needle (6), the needle (6) and the inner wall of the seal (5) are of complementary shape,
- immediately upstream of the additional hole, the needle (6) and the inner wall of the seal (5) do not have a complementary shape.

4. Connector according to claim 3, **characterised in that** the needle (6) and the inner wall of the seal (5) are of complementary shape on only part of the distance separating said hole from the distal end of the needle.

5. Connector according to one of claims 3 or 4, **characterised in that** the wall of the part of the seal whose shape complements that of the needle (6) is stiffer than that of the rest of the seal.

6. Connector according to one of claims 3 to 5, **characterised in that**:
- the inner wall of the resilient seal (5) has a generally conical shape over its entire length,
- immediately upstream of the additional hole (6b), the needle (6) has a cylindrical outer section.

7. Connector according to claim 6, **characterized in that** immediately upstream of the additional hole (6b), the needle (6) has a first part with a cylindrical outer section (6d) and a second conical part with an outer section (6e) that increases towards the base of the needle.

8. Connector according to one of claims 1 or 2, **characterised in that**:
- the resilient seal (5) has on its inner wall a first peripheral pad (5b 1) adapted to close at least one additional hole (6b),
- immediately downstream of the additional hole (6b), on at least part of the distance separating said hole from the distal end of the needle (6), the needle and the inner wall of the seal (5) are of non-complementary shape and demarcate a rigid, sealed cavity (9),
- immediately downstream of the additional orifice, the needle has a peripheral edge with a section substantially identical or almost identical to that of the cavity.

9. Connector according to claim 8, **characterised in that** immediately downstream of the first peripheral pad (5b 1), the inner wall of the seal (5) has a tubular portion demarcated downstream by a second peripheral bead (5b) held in tight contact with the needle (6).

10. Connector according to one of claims 8 or 9, **characterised in that** the wall of the tubular portion is more rigid than the rest of the seal.

11. Connector according to one of the preceding claims, **characterised in that** the wall of the seal upstream and downstream of the cavity is in the form of a succession of pads giving the parts of the seal identical rigidity downstream and upstream of the cavity.

12. Connector according to one of the preceding claims, **characterised in that** there are two additional holes which are positioned opposite one another.
